# EUROPEAN PATENT SPECIFICATION

(11) **EP 1 842 502 B1**
(45) Date of publication and mention of the grant of the patent: **09.03.2011**
(21) Application number: 07006985.1
(22) Date of filing: 03.04.2007
(51) Int. Cl.: A61B 17/32

(54) **Ultrasonic operating apparatus**
Ultraschalloperationsvorrichtung
Appareil à fonctionnement à ultrasons

(30) Priority: 06.04.2006 JP 2006105397
(43) Date of publication of application: 10.10.2007
(73) Proprietor: OLYMPUS MEDICAL SYSTEMS CORP., Shibuya-ku Tokyo 151-0072 (JP)
(72) Inventor: Okabe, Hiroshi, Hachioji-shi Tokyo 192-8512 (JP); Murakami, Eiji, Hachioji-shi Tokyo 192-8512 (JP); Ichihashi, Hiroshi, Hachioji-shi Tokyo 192-8512 (JP); Imoto, Masaru, Hachioji-shi Tokyo 192-8512 (JP); Oshida, Masami, Hachioji-shi Tokyo 192-8512 (JP)
(74) Representative: von Hellfeld, Axel

(56) References cited:
- JP-A- 2004 337 187
- US-A1- 2004 116 952

## Description

The present invention relates to a medical ultrasonic operating apparatus having a handpiece for medical operation by using ultrasonic oscillation.

A conventional ultrasonic operating apparatus is disclosed by Jpn. Pat. Appln. KOKAI Publication No. 2004-337187 (patent document 1). The apparatus described in this document comprises a main unit of a medical ultrasonic operating apparatus, a handpiece for ultrasonic operation connected to the main unit, and a footswitch. The apparatus is configured to start ultrasonic oscillation by stepping the footswitch.

The handpiece for ultrasonic operation described in the above patent document is provided with an electrode connected to a high-frequency power supply. When using the ultrasonic operating apparatus, apply a distal end of an ultrasonic probe incorporated in the handpiece for ultrasonic operation to a patient. In this state, medical operation using ultrasonic oscillation can be made, and coagulation of a living tissue and stop of bleeding can be made by supplying a high-frequency current to the electrode from a high-frequency source.

The above patent document 1 discloses a configuration wherein a hand switch is provided to start ultrasonic oscillation when detecting the operator's gripping of the handpiece handle for ultrasonic operation, and the operation of the handpiece is prohibited until ultrasonic oscillation and supply of high-frequency current become possible. This prevents accidental ultrasonic oscillation to a patient, and prohibits supply of high-frequency current under unnecessary conditions.

In a handpiece of a conventional ultrasonic operating apparatus, an operating instruction is mostly given from a footswitch. However, when the operation time is long, stepping a footswitch is tiring and troublesome. A footswitch may be replaced by a hand switch in some cases. In such a case, when making a delicate operation by using a handpiece for ultrasonic operation, the operation of the hand switch may cause deflection of a distal end of an ultrasonic probe. Therefore, if an ultrasonic oscillation can be automatically started when a living tissue is certainly grasped by the operation of closing a grasping part of a handpiece, it is desirable as an example of ideal operation form and demanded to realize.

Further, in the apparatus of the patent document 1, the structure of an ultrasonic oscillation control mechanism incorporated in the handle of the handpiece for ultrasonic operation is relatively complicated. This makes cleaning of the handpiece troublesome. It is thus necessary to realize technology to automatically oscillate an ultrasonic wave when a living tissue is certainly grasped by a handpiece for ultrasonic operation.

Document US 2004/0116952 A1 concerns surgical ultrasonic operating apparatuses. In one embodiment, an ultrasonic operating apparatus comprising movable jaws and means for generating ultrasonic oscillation is disclosed. The jaws may be operated by means of a fixed handle and a movable handle. When moving the movable handle towards the fixed handle, a switch for activating the generation of ultrasonic oscillation is activated.

The present invention has been made in the above circumstances and concerns an ultrasonic operating apparatus having the features of claim 1. It is an object of the invention to provide an ultrasonic operating apparatus, which can simplify the whole apparatus configuration, instruct to output oscillation energy, and output oscillation energy in the state that a living tissue is certainly grasped.

According to an embodiment of the invention, there is provided an ultrasonic operating apparatus comprising an ultrasonic oscillator to generate ultrasonic oscillation; an oscillation transmitting member for transmitting ultrasonic oscillation, which is made by a bar-like body having a distal end and a proximal end, the proximal end connected to the ultrasonic oscillator; a sheath which is made by a cylindrical body having a distal end and a proximal end, and inserted onto the oscillation transmitting member; a jaw which is provided at the distal end of the sheath, and grasps a living tissue in a clearance to the distal end of the oscillation transmitting member; a control unit which is provided at the proximal end of the sheath, and opens/closes the jaw with respect to the distal end of the oscillation transmitting member; and an instruction unit which detects that the jaw is closed by the control unit, and instructs the ultrasonic oscillator to output oscillation energy to generate ultrasonic oscillation when the jaw is closed.

The ultrasonic oscillator is driven by the instruction to output oscillation energy when the instruction unit detects the closure of the jaw by the control unit. The ultrasonic oscillation from the ultrasonic oscillator is transmitted to the oscillation transmitting member, and transmitted to the distal end of the oscillation transmitting member through the oscillation transmitting member. Interlocking with the jaw closing operation by the control unit, a living tissue is cut and coagulated by the oscillation energy while being grasped between the jaw and the distal end of the oscillation transmitting member.

Preferably, the control unit has a movable operation part to move to open/close the jaw, and the instruction unit has a detector to detect the closed state of the jaw by the amount of movement of the movable operation part.

In the above configuration, the closed state of the jaw is detected by the instruction unit by the amount of movement of the movable operation part.

Preferably, the control unit has a selector to change the largeness of ultrasonic oscillation generated by the ultrasonic oscillator.

In the above configuration, the largeness of ultrasonic oscillation generated by the ultrasonic oscillator is changed by the selector of the control unit.

According to another embodiment of the invention, there is provided an ultrasonic operation apparatus comprising a medical ultrasonic handpiece to cut and coagulate by using ultrasonic oscillation, the handpiece comprising an ultrasonic oscillator to generate ultrasonic oscillation; an oscillation transmitting member for transmitting ultrasonic oscillation, which has a proximal end connected to the ultrasonic oscillator; a grasping unit held openable/closable in a clearance to the distal end of the oscillation transmitting member; a grasping control unit which opens/closes the grasping unit, and grasps a living tissue between the grasping unit and the distal end of the oscillation transmitting member by closing the grasping unit with respect to the distal end of the oscillation transmitting member; and an instruction unit which corresponds to the states of the grasping control unit, and instructs to output oscillation energy from the ultrasonic oscillator in the state that a living tissue is grasped between the grasping unit and the distal end of the oscillation transmitting member.

In the above configuration, when using the handpiece, the ultrasonic oscillator is driven by the instruction to output oscillation energy when the instruction unit detects the closure of the grasping unit by the grasping control unit. The ultrasonic oscillation from the ultrasonic oscillator is transmitted to the oscillation transmitting member, and transmitted to the distal end of the oscillation transmitting member through the oscillation transmitting member. Interlocking with the grasping unit closing operation by the grasping control unit, a living tissue is cut and coagulated by the oscillation energy while being grasped between the grasping unit and the distal end of the oscillation transmitting member.

Preferably, the grasping control unit has a movable operation part to move to open/close the grasping unit, and the instruction unit gives the output instruction in the state that the grasping control unit is operated to a position to move the grasping unit to the closed state to grasp the living tissue between the grasping unit and the distal end of the oscillation transmitting member.

In the above configuration, the instruction unit gives an output instruction in the state that the movable part of the grasping control unit is operated to the position to move the grasping unit to the closed state to grasp a living tissue between the grasping unit and the distal end of the oscillation transmitting member.

The invention can be more fully understood from the following detailed description when taken in conjunction with the accompanying drawings, in which:
FIG. 1 is a side view showing a diagrammatic sketch of an ultrasonic surgical instrument according to a first embodiment of the invention;
FIG. 2A is a perspective view of an essential part of a fixed handle showing the mounting state of a switch for changing the output of the ultrasonic surgical instrument of the first embodiment;
FIG. 2B is a wiring diagram of a switch for changing the output of the ultrasonic surgical instrument of the first embodiment;
FIG. 3A is a perspective view of an essential part of a fixed handle showing the mounting state of a switch for changing the output of an ultrasonic surgical instrument according to a second embodiment;
FIG. 3B is a wiring diagram of a switch for changing the output of the ultrasonic surgical instrument of the second embodiment;
FIG. 4A is a perspective view of an essential part of a fixed handle showing the mounting state of a switch for changing the output of an ultrasonic surgical instrument according to a third embodiment;
FIG. 4B is a wiring diagram of a switch for changing the output of the ultrasonic surgical instrument of the third embodiment;
FIG. 5 is a view showing a diagrammatic sketch of an ultrasonic surgical instrument according to a fourth embodiment of the invention;
FIG. 6 is a side view showing a fixed handle of an ultrasonic surgical instrument of the fourth embodiment;
FIG. 7 is a side view showing a fixed handle of an ultrasonic surgical instrument according to a fifth embodiment of the invention;
FIG. 8 is a side view showing a fixed handle of an ultrasonic surgical instrument according to a sixth embodiment of the invention;
FIG. 9 is a side view showing a fixed handle of an ultrasonic surgical instrument according to a seventh embodiment of the invention;
FIG. 10 is a side view showing a fixed handle of an ultrasonic surgical instrument according to an eighth embodiment of the invention; and
FIG. 11 is a longitudinal sectional view of an essential part of an ultrasonic surgical instrument according to a ninth embodiment of the invention.

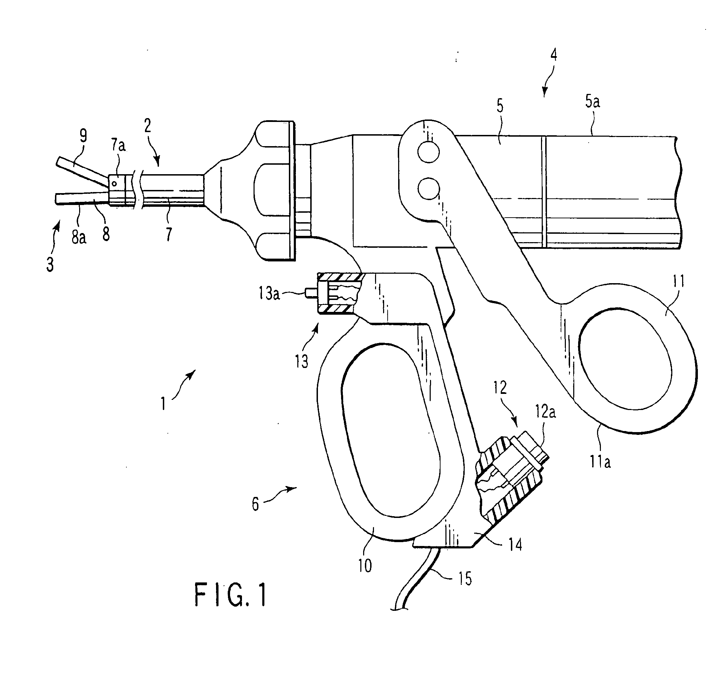

Merely figures 1-4 show the invention.

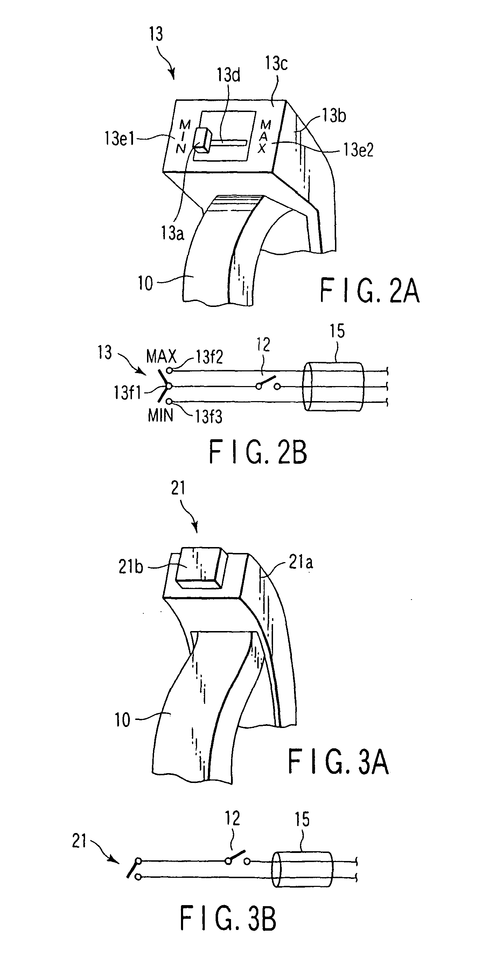

A first embodiment of the invention will be explained hereinafter with reference to FIG. 1 and FIGS. 2A and 2B. FIG. 1 shows a handpiece 1 of an ultrasonic surgical instrument as an ultrasonic operating apparatus of this embodiment. The handpiece 1 has a slender insertion section 2, a surgical unit 3 provided at the distal end of the insertion section 2, and a operation section 4 near at hand connected to the proximal end of the insertion section 2.

The operation section 4 is provided with a main body 5 and an operation handle 6. The main body 5 is connected to an ultrasonic oscillator 5a to generate an ultrasonic wave. The operation handle 6 operates the surgical unit 3.

The insertion section 2 has a pipe-like sheath 7, and an oscillation transmitting member 8. The oscillation transmitting member 8 is inserted into the sheath 7. The proximal end of the oscillation transmitting member 8 is connected to the ultrasonic oscillator 5a of the main body 5. The distal end 8a of the oscillation transmitting member 8 is exposed to the distal end of the sheath 7 of the insertion section 2. Ultrasonic oscillation output from the ultrasonic oscillator 5a is transmitted to the surgical unit 3 through the oscillation transmitting member 8.

The surgical unit 3 has a distal end 8a of the oscillation transmitting member 8 and a jaw 9 for cutting and coagulation. The jaw 9 has a pivotal support part in the proximal end. The pivotal support part of the jaw 9 is pivotally supported by a support member 7a. The jaw 9 is driven rotatably about the pivotal support part. In this time, the operation of moving the jaw 9 close to the distal end 8a of the oscillation transmitting member 8 is a closing operation, and the operation of moving the jaw 9 away from the distal end 8a of the oscillation transmitting member 8 is an opening operation. When the jaw 9 is closed, a living tissue is grasped between the jaw 9 and the distal end 8a of the oscillation transmitting member 8.

The operation handle 6 of the operation section 4 has a fixed handle 10 and a movable handle 11. The movable handle 11 is connected to a proximal end of a not-shown operation rod. The operation rod is inserted into the sheath 7 of the insertion section 2 to advance and retreat in the axial direction. The distal end of the operation rod is connected to the proximal end of the jaw 9. As the movable handle 11 of the operation section 4 is rotationally moved, the not-shown operation rod advances in the axial direction, and the jaw 9 is rotationally moved interlocking with the advance/retreat movement of the operation rod. By the rotational movement of the jaw 9, the jaw 9 is opened/closed with respect to the distal end 8a of the oscillation transmitting member 8.

The fixed handle 10 of the operation section 4 is provided with an open/close detection switch (indicator) 12, an output selector switch 13, and a switch mounting member 14. The output selector switch 13 is provided in the upper part of the fixed handle 10 (close to the part connected to the main body 5) as shown in FIG. 2A. The switch 13 is a slide type switch having a switch main body 13b and a slide lever 13a. The slide lever 13a of the output selector switch 13 is arranged forward facing to the distal end (the surgical unit 3).

The switch main body 13b has a front panel 13c. The front panel 13c has a guide groove 13d extending in the lateral direction. The slide lever 13a of the output selector switch 13 is moved in the lateral direction along the guide groove 13d in FIG. 2A. The front panel 13c of the switch main body 13b is given a MIN position mark 13e1 indicating a rated output at the left end of the guide groove 13d, and a MAX position mark 13e2 indicating a maximum output at the right end of the groove.

The output selector switch 13 functions to change an output signal of a power supply by changing connection of a signal conductor in an electric cable 15 by sliding the slide lever 13a. The output of the ultrasonic oscillator 5a is changeable in two steps of MAX and MIN by the switch 13. When the slide lever 13a is moved to the MAX position 13e2 at the right end in FIG. 2A, the output of the ultrasonic oscillator 5a is changed to a maximum output.

The open/close detection switch 12 is provided in the lower part of the fixed handle 10 and inclined upward to the movable handle 11, as shown in FIG. 1. The open/close detection switch 12 is a pushbutton tact switch. A pushbutton 12a of the open/close detection switch 12 is placed at a position to contact the end face 11a of the movable handle 11, when the movable handle 11 is moved close to the fixed handle 10. When the movable handle 11 is moved in the closing direction (coming close to the jaw 9), the end face 11a of the movable handle 11 presses the pushbutton 12a of the open/close detection switch 12, and brings the signal conductor into conduction. When the movable handle 11 is moved in the direction of separating from the fixed handle 10 and the end face 11a of the movable handle 11 is separated from the pushbutton 12a of the open/close detection switch 12, the tact switch is automatically turned off to shut off a signal.

One end of the electric cable 15 is connected to the switch mounting member 14. The other end of the electric cable 15 is connected to a not-shown power supply of the ultrasonic operating apparatus. A signal conductor in the electric cable 15 is wired to the open/close detection switch 12 and output selector switch 13, as shown in FIG. 2B. The output selector switch 13 is provided at one end of the signal conductor in the electric cable 15. The output selector switch 13 has a common terminal 13f1 and two switching terminals (MAX terminal 13f2 and MIN terminal 13f3). When the slide lever 13a is moved, one of the MAX terminal 13f2 and MIN terminal 13f3 is connected to the common terminal 13f1. The open/close detection switch 12 is provided on the way of the signal conductor connected to the common terminal 13f1. The open/close detection switch 12 switches on/off the output signal from the power supply, and the output selector switch 13 switches the output signal of the power supply, thereby controlling an ultrasonic output.

Next, the function of the above configuration will be explained. When the handpiece 1 of the ultrasonic operating apparatus of this embodiment is used, the operation handle 6 of the control unit is operated to open and close. As the operation handle 6 is operated to open or close, a not-shown operation rod advances or retreats in the axial direction, and the jaw 9 moves rotationally interlocking with the advance/retreat of the operation rod. By the rotational movement, the jaw 9 is opened or closed with respect to the distal end 8a of the oscillation transmitting member 8. In this time, the open/close detection switch 12 is turned on/off by the rotational movement of the movable handle 11 in the direction of coming close to the fixed handle 10, thereby turning on/off of driving of the ultrasonic oscillator 5a is controlled.

One of MAX and MIN outputs is previously selected and then changed by the slide lever 13a of the output selector switch 13. Therefore, the connection of the signal conductor in the electric cable 15 is switched, and the output signal of the power supply is switched. The output may be switched by operating the slide lever 13a of the output selector switch 13 if necessary during operation of the ultrasonic operation apparatus.

When the movable handle 11 of the operation handle 6 of the operation section 4 is held open separated from the fixed handle 10, the jaw 9 is held open separated from the distal end 8a of the oscillation transmitting member 8. In this time, the open/close detection switch 12 is kept off, and the ultrasonic oscillator 5a in the handpiece 1 is held not driven.

When the movable handle 11 of the operation handle 6 of the operation section 4 is rotationally moved close to the fixed handle 10 (in the closing direction), the not-shown operation rod is moved backward, and the jaw 9 is moved in the closing direction. In this time, the jaw 9 is closed to the distal end 8a of the oscillation transmitting member 8, and a living tissue is grasped between the jaw 9 and the distal end 8a of the oscillation transmitting member 8.

When the movable handle 11 is moved in the closing direction further close to the fixed handle 10, the end face 11a of the movable handle 11 presses the open/close detection switch 12. The open/close detection switch 12 is turned on by this depression, the signal conductor of the electric cable 15 becomes conductive, and the ultrasonic oscillator 5a is driven. Power from the main power supply is converted to oscillation energy in the ultrasonic oscillator 5a, and the ultrasonic energy is amplified and transmitted to the distal end 8a of the oscillation transmitting member 8. In this time, as the jaw 9 presses (holds) a living tissue to the distal end 8a of the oscillation transmitting member 8, the surgical unit 3 cuts and coagulates a living tissue.

When the movable handle 11 of the operation handle 6 is moved away from the fixed handle 10 (in the opening direction), the not-shown operation rod advances, and the jaw 9 is moved in the opening direction and separated from the distal end 8a of the oscillation transmitting member 8. When the movable handle 11 is opened, the end face 11a of the movable handle 11 is separated from the open/close detection switch 12. As the open/close detection switch 12 is released, the signal conductor becomes non-conductive, and the tact switch of the open/close detection switch 12 is automatically turned off to shut off the signal. Therefore, the ultrasonic oscillator 5a is stopped.

The above configuration provides the following effects. In the handpiece 1 of the medical ultrasonic surgical instrument of this embodiment, the open/close detection switch 12 is provided in the lower part of the fixed handle 10 and inclined upward to the movable handle 11. When the movable handle 11 of the operation handle 6 is moved in the closing direction with respect to the fixed handle 10, the end face 11a of the movable handle 11 contacts the open/close detection switch 12. While the jaw 9 is being closed, the switch 12 detects the jaw 9 closed, and instructs the ultrasonic oscillator 5a to output oscillation energy to generate ultrasonic oscillation. Therefore, ultrasonic energy can be automatically output simply by gripping the movable handle 11 of the operation handle 6. This eliminates the necessity of operating a footswitch or a hand switch to turn on/off the ultrasonic oscillator 5a as in a conventional common medical ultrasonic handpiece, and increases the operability of the handpiece 1 compared with the prior art. Further, as the output selector switch 13 freely changes a setting of output from the ultrasonic oscillator 5a, the operability of the handpiece 1 can be increased furthermore.

In this embodiment, by gripping the movable handle 11 of the operation handle 6, the jaw 9 presses a living tissue to the distal end 8a of the oscillation transmitting member 8, and when a living tissue is securely grasped between the jaw 9 and the distal end 8a of the oscillation transmitting member 8, the ultrasonic oscillator 5a can be automatically operated to generate ultrasonic oscillation. Therefore, it is possible to instruct to turn on/off the oscillation energy output according to the states of the movable handle 11 of the control handle 6. This realizes an example of ideal operation form. Namely, a desired ultrasonic operation is possible only when a living tissue is completely grasped between the jaw 9 and the distal end 8a of the oscillation transmitting member 8. This provides the effect of mechanically and certainly preventing an error output.

Further, the jaw 9 is generally provided with a soft member (e.g. Teflon [registered trademark]) on the surface to contact the distal end 8a of the oscillation transmitting member 8. This prevents breaking of the distal end 8a of the oscillation transmitting member 8 caused by the oscillation, when the jaw 9 contacts the distal end 8a of the oscillation transmitting member 8 during ultrasonic oscillation. In the configuration of this embodiment, when the gripping of the movable handle 11 is released after cutting a living tissue, the output is automatically stopped. This prevents output of unnecessary ultrasonic oscillation energy. As a result, wear of the soft member provided in the jaw 9 to contact the distal end 8a of the oscillation transmitting member 8 during ultrasonic oscillation, is prevented. This provides the effect of increasing the durability of the apparatus.

FIGS. 3A and 3B show a second embodiment of the invention. In this embodiment, the configuration of the handpiece 1 of the medical ultrasonic surgical instrument of the first embodiment (refer to FIG. 1 and FIGS. 2A and 2B) is modified.

Namely, in this embodiment, the output selector switch 13 of the first embodiment provided in the fixed handle 10 of the operation section 4 is replaced by an output switch 21, as shown in FIG. 3A. The output switch 21 is a tact switch having a switch main body 21a and a pushbutton 21b provided to project to and sink in the end face of the switch main body 21a. The switch 21 is usually held by a not-shown spring member at a fixed position where the pushbutton 21b is projected from the end face of the switch main body 21a. In this time, the output switch 21 is kept off.

The output switch 21, open/close detection switch 12 and electric cable 15 are wired as shown in FIG. 3B. In the circuit shown here, a signal flows when the pushbutton 21b of the output switch 21 is pressed in the state that the open/close detection switch 12 is turned on.

Next, the function of the above configuration will be explained. When using the handpiece 1 of the ultrasonic operating apparatus of this embodiment, move the movable handle 11 in the closing direction (the direction of closing the jaw 9). The end face 11a of the movable handle 11 presses the pushbutton 12a of the open/close detection switch 12. Then, the open/close detection switch 12 is turned on. When the pushbutton 21b of the output switch 21 is further pressed in this state, the signal conductor becomes conductive. In this time, while the pushbutton 21b of the output switch 21 is being pressed, the signal conductor is held conductive.

When the depression of the pushbutton 21b of the output switch 21 is released and the pushbutton 12a is returned to the fixed position projecting from the end face of the switch main body 21a, the tact switch is automatically turned off and the signal is shut off. Thereafter, when the movable handle 11 is moved away from the fixed handle 10 and the end face 11a of the movable handle 11 is separated from the pushbutton 12a of the open/close detection switch 12, the tact switch of the open/close detection switch 12 is automatically turned off.

This embodiment provides the following effects. In the handpiece 1 of the medical ultrasonic surgical instrument of this embodiment, the output switch 21 and open/close detection switch 12 are provided in the fixed handle 10 of the operation section 4, forming a circuit which flows a signal when the pushbutton 21b of the output switch 21 is pressed in the state that the open/close detection switch 12 is turned on. Therefore, the ultrasonic oscillator 5a is not operated until the pushbutton 21b of the output switch 21 is depressed. This provides the effect of securely preventing an error output.

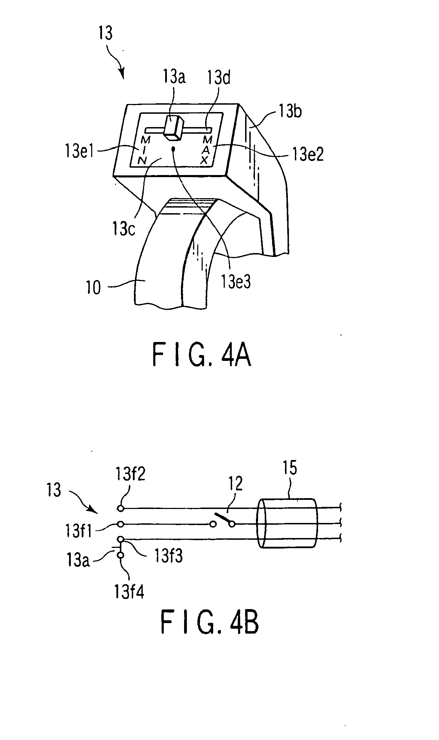

Further, when the gripping of the movable handle 11 is released after cutting a living tissue, the open/close detection switch 12 is turned off. Therefore, even if the depression of the pushbutton 21b of the output switch 21 is not released (forget to release), the output is automatically stopped, and output of unnecessary ultrasonic oscillation energy is prevented. As a result, wear of the soft member provided in the jaw 9 to contact the distal end 8a of the oscillation transmitting member 8 during ultrasonic oscillation, is prevented. This also provides the effect of increasing the durability of the apparatus. FIGS. 4A and 4B show a third embodiment of the invention. In this embodiment, the configuration of the output selector switch 13 of the handpiece 1 of the medical ultrasonic surgical instrument of the first embodiment (refer to FIG. 1 and FIGS. 2A and 2B) is modified.

Namely, in this embodiment, the output selector switch 13 is designed to change the output of the ultrasonic oscillator 5a in three steps of MAX, MIN, and no output, as shown in FIG. 4A. Here, the front panel 13c of the switch main body 13b of the output selector switch 13 is given a MIN position mark 13e1 indicating a rated output at the left end of the guide groove 13d, a MAX position mark 13e2 indicating a maximum output at the right end of the groove, and a middle position mark 13e3, for example, a black circle to select no output at the middle of the groove.

The signal conductor in the electric cable 15 is wired to the open/close detection switch 12 and output selector switch 13, as shown in FIG. 4B. The output selector switch 13 is provided at one end of the signal conductor in the electric cable 15. The output selector switch 13 has a common terminal 13f1 and three switching terminals (MAX terminal 13f2, MIN terminal 13f3, and no connection terminal 13f4). When the operation lever 13a is moved to the MAX position 13e2, the MAX terminal 13f3 and common terminal 13f1 are connected to provide a maximum output. When the operation lever 13a is moved to the MIN position 13e1, the MIN terminal 13f3 and common terminal 13f1 are connected to provide a rated output. When the operation lever 13a is moved to the middle position 13e3, for example, a black circle, the no connection terminal 13f4 is connected to provide no output.

The open/close detection switch 12 is provided on the way of the signal conductor connected to the common terminal 13f1. The open/close detection switch 12 switches on/off the output signal from the power supply, and the output selector switch 13 switches the output signal of the power supply, thereby controlling an ultrasonic output.

Next, the function of the above configuration will be explained. When operating the handpiece 1 of the ultrasonic operating apparatus of this embodiment, the operation is almost the same as in the first embodiment. When operating the output selector switch 13, the operation lever 13a can be selectively moved to the positions of MAX 13e2, MIN 13e1, and middle 13e3, for example, a black circle, thereby selecting one of MAX, MIN and no output.

When the operation lever 13a is moved to the MAX position 13e2, the MAX terminal 13f2 and common terminal 13f1 are connected to provide a maximum output. When the operation lever 13a is moved to the MIN position 13e1, the MIN terminal 13f3 and common terminal 13f1 are connected to provide a rated output.

When the operation lever 13a is moved to the middle position 13e3, the ultrasonic oscillator 5a is kept off. The signal conductor of the electric cable 15 does not form a circuit in this state, and the ultrasonic oscillator 5a is kept off and does not output an ultrasonic wave even if the movable handle 11 of the operation handle 6 is moved in the closing direction to the fixed handle 10.

The above configuration provides the following effects. In the handpiece 1 of the medical ultrasonic surgical instrument of this embodiment, by gripping the movable handle 11 of the operation handle 6 in the state that the operation lever 13a of the output selector switch 13 is previously moved to one of the MAX position 132e and MIN position 13e1, the ultrasonic oscillator 5a can be automatically operated when a living tissue is pressed to the distal end 8a of the oscillation transmitting member 8 by the jaw 9 and certainly grasped between the jaw 9 and the distal end 8a of the oscillation transmitting member 8. Therefore, as in the first embodiment, an instruction can be given to switch on/off oscillation energy output according to the states of the movable handle 11. This eliminates the necessity of operating a footswitch or a hand switch to turn on/off the ultrasonic oscillator 5a as in a conventional common medical ultrasonic handpiece, and increases the operability of the handpiece 1 compared with the prior art.

Further, as the output selector switch 13 freely changes a setting of output from the ultrasonic oscillator 5a, the operability of the handpiece 1 can be increased furthermore. As in the first embodiment, on/off of oscillation energy can be controlled according to the states of the movable handle 11. This securely and mechanically prevents an error output, and prevents output of unnecessary ultrasonic oscillation energy, and wear of the soft member provided in the jaw 9 to contact the distal end 8a of the oscillation transmitting member 8 during ultrasonic oscillation. As a result, the durability of the apparatus can be increased. Further, the slide lever 13a of the output selector switch 13 has the middle position 13e3. Therefore, the ultrasonic oscillator 5a is not operated even if the movable handle 11 of the operation handle 6 is moved in the closing direction to the fixed handle 10. This securely and mechanically prevents an error output. The apparatus is not worn by an unnecessary ultrasonic oscillation energy output. In this state, the handpiece 1 can be used simply as a biopsy forceps just for grasping without outputting an ultrasonic wave.

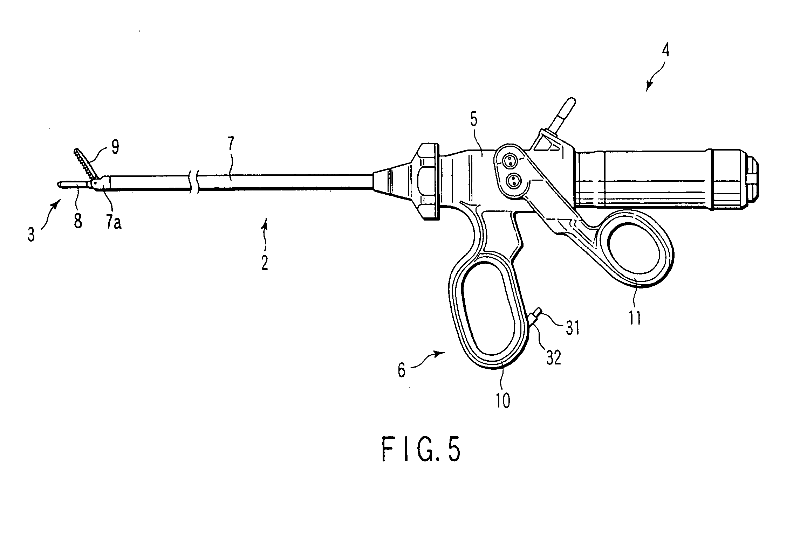

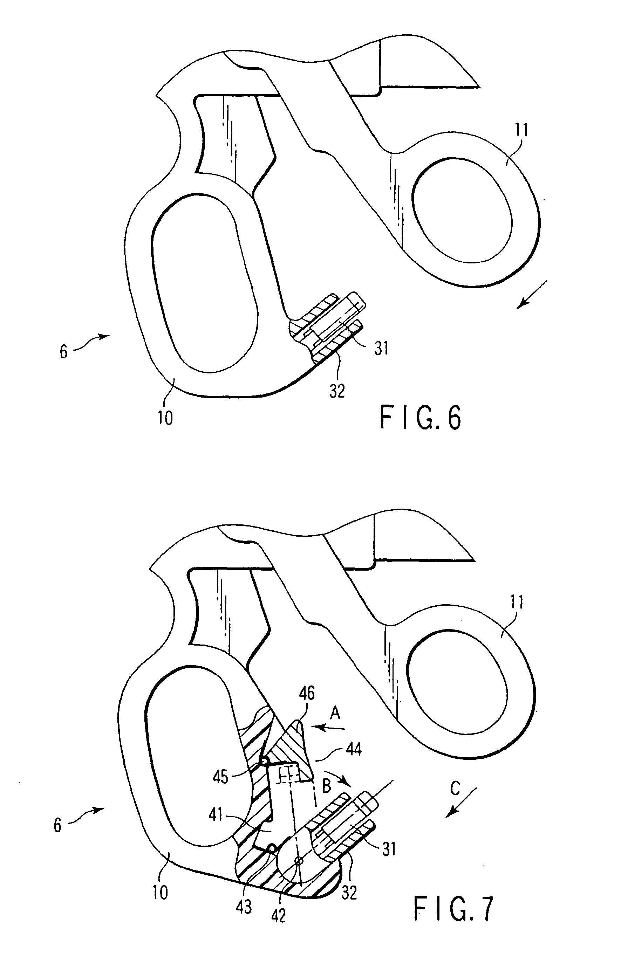

FIG. 5 and FIG. 6 show a fourth embodiment of the invention. FIG. 5 shows a handpiece 1 of a medical ultrasonic surgical instrument of this embodiment. In FIG. 5 and FIG. 6, the same parts of those of the handpiece 1 of the ultrasonic surgical instrument of the first embodiment (refer to FIG. 1 and FIG. 2 and 2B) are given the same reference numerals, and detailed explanation is omitted.

In this embodiment, an output switch 31 for starting ultrasonic output is provided in the fixed handle 10 of the operation handle 6 of the handpiece 1. In the lower end part of the fixed handle 10 in FIG. 6, a cylindrical switch holder 32 is provided projecting in the direction opposite to the movable handle 11. The output switch 31 is inserted into the switch holder 32. The end portion of the output switch 31 is exposed to the outside of the switch holder 32. Further, in this embodiment, the output switch 31 is arranged at a position near the end of the moving path of the movable handle 11 when moving in the closing direction to the fixed handle 10, as indicated by an arrow in FIG. 6.

The function of the above configuration will be explained. In this embodiment, when operating the operation handle 6 of the handpiece 1, the jaw 9 is closed to the distal end 8a of the oscillation transmitting member 8 by moving the movable handle 11 close to the fixed handle 10, as indicated by an arrow in FIG. 6. Therefore, a living tissue is grasped between the jaw 9 and the distal end 8a of the oscillation transmitting member 8. In this time, when a living tissue is completely grasped by the surgical unit 3, the output switch 31 of the fixed handle 10 is pressed by the movable handle 11. The output switch 31 is turned on, and an ultrasonic wave is output.

The above configuration provides an auto output switch system, which outputs an ultrasonic wave only by one operation of moving the movable handle 11 by interlocking an operation of the movable handle 11 necessary for opening/closing the jaw 9 of the surgical unit 3 and an output means for starting ultrasonic output. This eliminates operation of other output switches such as a footswitch, and increases the operability of the handpiece 1 compared with the prior art.

Further, the output switch 31 is arranged at the position pressed by the movable handle 11 in the state that the surgical unit 3 completely grasps a living tissue. Therefore, as in the first embodiment, an error output can be mechanically prevented, and wear of the apparatus by an unnecessary output of ultrasonic oscillation energy can be prevented.

FIG. 7 shows a fifth embodiment of the invention. In this embodiment, the configuration of the fixed handle 10 of the operation handle 6 of the handpiece 1 of the fourth embodiment (refer to FIG. 5 and FIG. 6) is modified.

Namely, in this embodiment, a switch housing cavity 41 is formed in the wall of the fixed handle 10 opposite to the movable handle 11. A rotation axis 42 of the switch holder 32 is provided at the lower end of the switch housing cavity 41 in FIG. 7. The proximal end of the switch holder 32 is pivotally supported rotatably about the rotation axis 42. The switch holder 32 is rotatable between the standby position housed in the switch housing cavity 41, as indicated by a virtual line in FIG. 7, and the operation position projected outside the switch housing cavity 41, as indicated by a solid line in FIG. 7.

A first spring member 43 is provided at the lower end of the switch housing cavity 41. The first spring member 43 energizes the switch holder 32 in the using direction. A lock member 44 is provided at the upper end of the switch housing cavity 41. The lock member 44 locks the switch holder 32 retracted to the standby position. The lock member 44 has a second spring member 45, and an operation knob 46. The second spring member 45 energizes the lock member 44 in the direction of locking/releasing the output switch 31 in the switch holder 32. The operation knob 46 is movable in the direction of releasing the lock member 44 from the output switch 31, against the spring force of the second spring member 45.

In this embodiment, the output switch 31 can be held at the standby position housed in the switch housing cavity 41. By releasably locking the lock member 44 with the output switch 31 in the switch holder 32, the output switch 31 in the switch holder 32 can be held in the switch housing cavity 41 of the fixed handle 10.

When the lock member 44 is moved by the operation knob 46 in the direction of releasing the lock with the output switch 31 as indicated by the arrow A in FIG. 7, the switch holder 32 is rotated to the using position projected outside from the switch housing cavity 41 by the force of the first spring member 43 as indicated by the arrow B in FIG. 7. In this state, the output switch 31 is exposed to the position opposite to the movable handle 11. Therefore, by moving the movable handle 11 close to the fixed handle 10 as indicated by the arrow C in FIG. 7, the output switch 31 of the fixed handle 10 is pressed by the movable handle 11, the output switch 31 is turned on, and output of ultrasonic wave can be started.

This embodiment provides the following effects. In the handpiece 1 of the medical ultrasonic surgical instrument of this embodiment, the output switch 31 provided in the fixed handle 10 can be housed in the switch housing cavity 41 of the fixed handle 10 as shown in FIG. 7. By holding the output switch 31 at the standby position housed in the switch housing cavity 41 of the fixed handle 10, the ultrasonic oscillator 5a can be held not driven even if the movable handle 11 is moved close to the fixed handle 10. Therefore, in this case, by moving the movable handle 11 close to the fixed handle 10, the ultrasonic oscillator can be held not driven even if the jaw 9 is closed to the distal end 8a of the oscillation transmitting member 8 and a living tissue is grasped between the jaw 9 and the distal end 8a of the oscillation transmitting member. In this state, the handpiece 1 can be used simply as a biopsy forceps.

By moving the lock member 44 in the direction of releasing from the output switch 31 by operating the operation knob 46, the switch holder 32 can be rotated to the using position projected outside the switch housing cavity 41. In this case, as in the fourth embodiment, by moving the movable handle 11 close to the fixed handle 10, the jaw 9 is closed to the distal end 8a of the oscillation transmitting member 8. When the jaw 9 and the distal end 8a of the oscillation transmitting member 8 grasp a living tissue, the movable handle 11 presses the output switch 31 of the fixed handle 10 to turn on the switch 31, and an ultrasonic wave is output. Therefore, in this embodiment, as the output switch 31 of the fixed handle 10 can be housed in the switch housing cavity 41 of the fixed handle 10, the operator can appropriately select an ultrasonic output in the output switch 31.

In this embodiment, the output switch 31 of the fixed handle 10 can be housed in the switch housing cavity 41. However, the output switch is not limited to this configuration. The movable handle 11 may be moved to a position not to press the output switch 31, when moved to the end position in the closing operation and the surgical unit 3 completely grasps a living tissue.

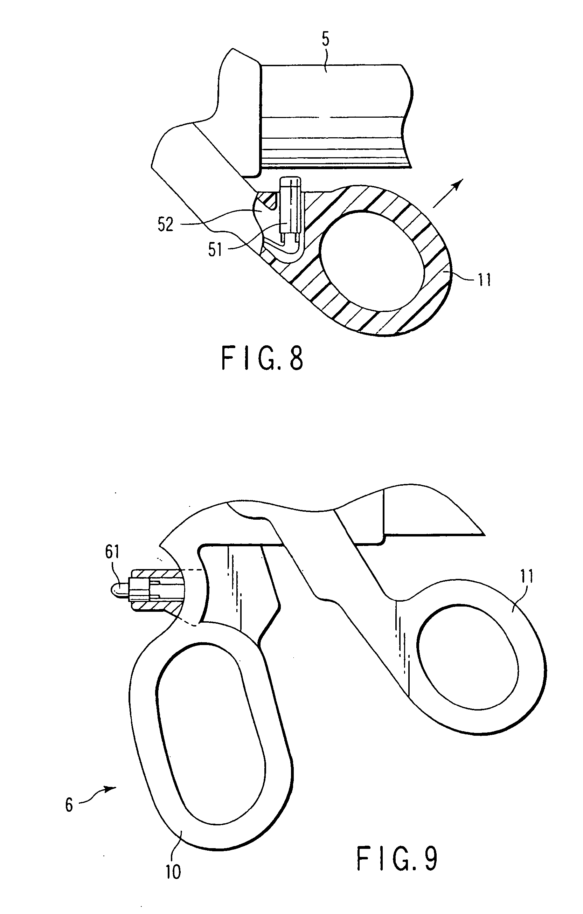

FIG. 8 shows a sixth embodiment of the invention. In this embodiment, the configuration of the operation handle 6 of the handpiece 1 of the fourth embodiment (refer to FIG. 5 and FIG. 6) is modified.

Namely, in this embodiment, an output switch 51 for starting ultrasonic output is provided in the movable handle 11 of the operation handle 6. Here, the movable handle 11 has a switch fitting hole 52 at a position separated and opposed to the control unit main body 5. The output switch 51 is fit in the switch fitting hole 52. The end portion of the output switch 51 is exposed to the outside of the switch fitting hole 52.

The function of the above configuration will be explained. In this embodiment, when the movable handle 11 of the operation handle 6 of the handpiece 1 is moved to the fixed handle 10 and the surgical unit 3 is completely opened, the output switch 51 provided in the movable handle 11 contacts the control unit main body 5 as indicated by an arrow in FIG. 8. The output switch 51 is pressed by the main body 5, and an ultrasonic wave is output.

The above configuration provides an auto output switch system, which outputs an ultrasonic wave only by one operation of moving the movable handle 11 by interlocking an operation of the movable handle 11 necessary for opening/closing the jaw 9 of the surgical unit 3 and an output means for starting ultrasonic output. This eliminates operation of other output switches such as a footswitch, and increases the operability of the handpiece 1 compared with the prior art.

Further, the output switch 51 is arranged at the position pressed by the control unit main body 5 in the state that the surgical unit 3 completely grasps a living tissue. Therefore, as in the first embodiment, an error output can be mechanically prevented, and wear of the apparatus by an unnecessary output of ultrasonic oscillation energy can be prevented.

FIG. 9 shows a seventh embodiment of the invention. In this embodiment, an output switch operation button 61 is provided at the front end of the fixed handle 10, instead of the output switch 31 of the fixed handle 10 in the fourth embodiment (refer to FIG. 5 and FIG. 6). In this case, by pressing the operation button 61, the output switch is turned on, and an ultrasonic wave is output.

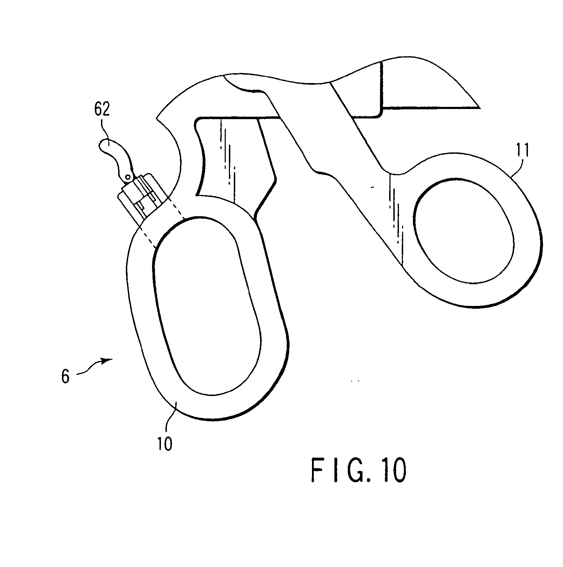

FIG. 10 shows an eighth embodiment of the invention. In this embodiment, an output switch operation lever 62 is provided at the front end of the fixed handle 10, instead of the output switch 31 of the fixed handle 10 in the fourth embodiment (refer to FIG. 5 and FIG. 6). In this case, by operating the operation lever 62, the output switch is turned on, and an ultrasonic wave is output.

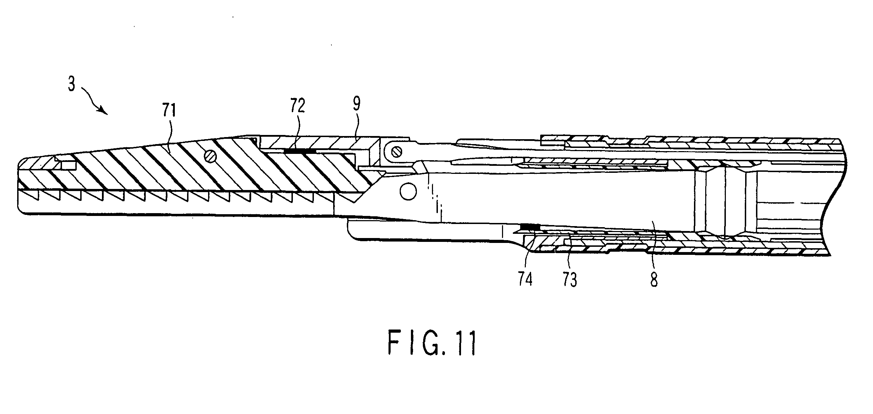

FIG. 11 shows a ninth embodiment of the invention. As described hereinbefore, an output switch is provided in the operation section 4 of the handpiece 1 in the first to eight embodiments, but an output means is not limited to this. The ninth embodiment shows an example of other configuration than that an output switch is provided in the operation section 4. The same parts as those of the handpiece 1 of the ultrasonic surgical instrument of the first embodiment are given the same reference numerals, and detailed description is omitted.

Namely, in this embodiment, a pressure sensor 72 is provided as a detection means between the jaw 9 and a grasping part 71 of the surgical unit 4 in FIG. 11. Therefore, when the jaw 9 and the distal end 8a of the oscillation transmitting member 8 grasp a living tissue, the pressure sensor 72 detects the pressure acting upon the oscillation transmitting member 8, and detects that a living tissue is completely grasped.

A deflection sensor 74 may be provided as a detection means between the oscillation transmitting member 8 and distal end cover 73, so that when the jaw 9 and the distal end 8a of the oscillation transmitting member 8 grasp a living tissue, the deflection sensor 74 detects the amount of deflection of the oscillation transmitting member 8, and detects that a living tissue is completely grasped. The pressure sensor 72 and deflection sensor 74 may be a switch type, and may be provided in any place that can be detected or a place to contact the oscillation transmitting member 8.

The function of the above configuration will be explained. When using the handpiece 1 of the ultrasonic operation apparatus of this embodiment, the pressure sensor 72 detects that the grasping part 71 of the surgical unit 3 completely grasps a living tissue by moving the movable handle 11, an output signal is received from a not-shown control means, and ultrasonic oscillation is started.

Otherwise, the deflection sensor 74 detects that the grasping part 71 completely grasps a living tissue by moving the movable handle 11, an output signal is received from a not-shown control means, and ultrasonic oscillation is started.

This embodiment provides the following effect. In the handpiece 1 of the medical ultrasonic surgical instrument of this embodiment, a handle operation for grasping a living tissue can be interlocked with an output means for starting ultrasonic oscillation, and ultrasonic oscillation can be started only by one handle operation. This eliminates operation of other output switches such as a footswitch, and provides an auto output switch system.

The invention is not limited to the aforementioned embodiments. For example, an output switch can be provided as an option in the handpiece 1. The structure is simple, and cleaning is easy. The invention may be embodied in other specific forms without departing from the scope of the claims.

The invention is effective in a technical field using a medical ultrasonic operating apparatus with a handpiece for ultrasonic operation, and in a field of manufacturing a medical ultrasonic operating apparatus with the handpiece for ultrasonic operation.

## Claims

1. An ultrasonic operating apparatus comprising:
an ultrasonic oscillator (5a) to generate ultrasonic oscillation;
an oscillation transmitting member (8) for transmitting ultrasonic oscillation, which is made by a bar-like body having a distal end and a proximal end, the proximal end connected to the ultrasonic oscillator (5a);
a sheath (7) which is made by a cylindrical body having a distal end and a proximal end, and inserted onto the oscillation transmitting member (8);
a jaw (9) which is provided at the distal end of the sheath (7), and may grasp a living tissue in a clearance to the distal end of the oscillation transmitting member (8);
a control unit (4) which is provided at the proximal end of the sheath (7) and has a fixed operation part (10) and a movable operation part (11), and opens/closes the jaw (9) with respect to the distal end of the oscillation transmitting member (8); and
an instruction unit (12, 31, 51) which detects that the jaw (9) is closed by the control unit (4), and instructs the ultrasonic oscillator (5a) to output oscillation energy to generate ultrasonic oscillation when the jaw (9) is closed **characterized by** a selector (13) to change largeness of ultrasonic oscillation generated by the ultrasonic oscillator (5a), wherein the selector (13) is provided at the fixed operation part (10).

2. The ultrasonic operating apparatus according to claim 1, **characterized in that** the instruction unit (12) has a detector (12a) to detect closure of the jaw (9) by the amount of movement of the movable operation part (11).

3. The ultrasonic operating apparatus according to claim 1, **characterized in that**:
the jaw (9) has a grasping part (71) which may contact with a living tissue; and
the instruction unit has a pressure sensor (72) serving as a detection means between the jaw (9) and the grasping part (71), the pressure sensor (72) being configured to detect a state where the living tissue is completely grasped by the grasping part (71).

4. The ultrasonic operating apparatus according to claim 1, **characterized in that** the sheath (7) has a deflection sensor (74) serving as a detection means between the oscillation transmitting member (8) and the distal end portion of the sheath (7), and when the jaw (9) and a distal end (8a) of the oscillation transmitting member (8) grasp a living tissue, the deflection sensor (74) detects an amount of deflection of the oscillation transmitting member (8), and detects that the living tissue is completely grasped.

## Patentansprüche

1. Ultraschallbetätigungsvorrichtung aufweisend:
einen Ultraschalloszillator (5a) zum Erzeugen von Ultraschalloszillation;
ein Oszillationsübertragungselement (8) zum Übertragen von Ultraschalloszillation, das aus einem stabartigen Körper gebildet ist, der ein Distalende und ein Proximalende aufweist, wobei das Proximalende mit dem Ultraschalloszillator (5a) verbunden ist;
eine Hülle (7), die durch einen zylindrischen Körper gebildet ist, der ein Distalende und ein Proximalende aufweist, und die über dem Oszillationsübertragungselement (8) angeordnet ist;
eine Backe (9), die an dem Distalende der Hülle (7) in einer Ausnehmung an dem Distalende des Oszillationsübertragungselements (8) vorgesehen ist, und die ein lebendes Gewebe greifen kann;
eine Steuerungseinheit (4), die an dem Proximalende der Hülle (7) vorgesehen ist und ein befestigtes Betätigungsteil (10) und ein bewegliches Betätigungsteil (11) aufweist, und welche die Backe (9) hinsichtlicht des Distalendes des Ozillationübertragungselements (8) öffnet/schließt; und
eine Befehlseinheit (12, 31, 51), die erfasst, dass die Backe (9) durch die Steuerungseinheit (4) geschlossen ist und den Ultraschalloszillator (5a) instruiert, Oszillationsenergie zur Erzeugung von Ultraschalloszillation auszugeben wenn die Backe (9) geschlossen ist,
**gekennzeichnet durch** einen Selektor (13) zum Ändern der Größe der **durch** den Ultraschalloszillator (5a) erzeugten Ultraschalloszillation, wobei der Selektor (13) an dem befestigten Betätigungsteil (10) vorgesehen ist.

2. Ultraschallbetätigungsvorrichtung nach Anspruch 1, **dadurch gekennzeichnet, dass** die Befehlseinheit (12) einen Detektor (12a) zum Erfassen der Schließung der Backe (9) durch den Bewegungsbetrag des beweglichen Betätigungsteils (11) aufweist.

3. Ultraschallbetätigungsvorrichtung nach Anspruch 1, **dadurch gekennzeichnet. dass**:
die Backe (9) ein Greifteil (71) aufweist, welches mit einem lebenden Gewebe in Kontakt treten kann; und
die Befehlseinheit einen Drucksensor (72) aufweist, der als Erfassungsmittel zwischen der Backe (9) und dem Greifteil (71) dient, wobei der Drucksensor (72) dazu eingerichtet ist, einen Zustand zu erfassen, bei dem das lebende Gewebe vollständig durch das Greifteil (71) gegriffen wird.

4. Ultraschallbetätigungsvorrichtung nach Anspruch 1, **dadurch gekennzeichnet, dass**
die Hülle (7) einen Ablenkungssensor (74) aufweist, der als Erfassungsmittel zwischen dem Oszillationsübertragungselement (8) und dem Distalendteil der Hülle (7) dient, wobei, wenn die Backe (9) und ein Distalende (8a) des Oszillationsübertragungselements (8) ein lebendes Gewebe greifen, der Ablenkungssensor (74) einen Betrag der Auslenkung des Oszillationsübertragungselements (8) erfasst, und erfasst, dass das lebende Gewebe vollständig gegriffen wird.

## Revendications

1. Appareil à fonctionnement à ultrasons comprenant :
un oscillateur à ultrasons (5a) pour générer une oscillation par ultrasons ;
un élément de transmission d'oscillation (8) destiné à transmettre une oscillation par ultrasons, qui est composé d'un corps en forme de barre comportant une extrémité distale et une extrémité proximale, l'extrémité proximale étant connectée à l'oscillateur à ultrasons (5a) ;
une gaine (7) qui est composée d'un corps cylindrique comportant une extrémité distale et une extrémité proximale, et insérée sur l'élément de transmission d'oscillation (8) ;
une mâchoire (9) qui est prévue au niveau de l'extrémité distale de la gaine (7), et qui peut saisir un tissu vivant dans un espace par rapport à l'extrémité distale de l'élément de transmission d'oscillation (8) ;
une unité de commande (4) qui est prévue au niveau de l'extrémité proximale de la gaine (7) et comporte une partie d'actionnement fixe (10) et une partie d'actionnement mobile (11), et qui ouvre/ferme la mâchoire (9) par rapport à l'extrémité distale de l'élément de transmission d'oscillation (8) ; et
une unité d'instruction (12, 31, 51) qui détecte que la mâchoire (9) est fermée par l'unité de commande (4), et délivre comme instruction à l'oscillateur à ultrasons (5a) de délivrer en sortie une énergie d'oscillation pour générer une oscillation par ultrasons lorsque la mâchoire (9) est fermée
**caractérisé par** un sélecteur (13) pour changer la largeur de l'oscillation par ultrasons générés par l'oscillateur à ultrasons (5a), dans lequel le sélecteur (13) est prévu au niveau de la partie d'actionnement fixe (10).

2. Appareil à fonctionnement à ultrasons selon la revendication 1, **caractérisé en ce que** l'unité d'instruction (12) comporte un détecteur (12a) pour détecter la fermeture de la mâchoire (9) de la quantité de mouvement de la partie d'actionnement mobile (11).

3. Appareil à fonctionnement à ultrasons selon la revendication 1, **caractérisé en ce que** :
la mâchoire (9) comporte une partie de préhension (71) qui peut entrer en contact avec un tissu vivant ; et
l'unité d'instruction comporte un capteur de pression (72) servant de moyen de détection entre la mâchoire (9) et la partie de préhension (71), le capteur de pression (72) étant configuré pour détecter un état dans lequel le tissu vivant est complètement saisi par la partie de préhension (71).

4. Appareil à fonctionnement à ultrasons selon la revendication 1, **caractérisé en ce que** la gaine (7) comporte un capteur de déviation (74) servant de moyen de détection entre l'élément de transmission d'oscillation (8) et la partie d'extrémité distale de la gaine (7), et lorsque la mâchoire (9) et une extrémité distale (8a) de l'élément de transmission d'oscillation (8) saisissent un tissu vivant, le capteur de déviation (74) détecte une quantité de déviation de l'élément de transmission d'oscillation (8), et détecte que le tissu vivant est complètement saisi.
